Europäisches Patentamt

European Patent Office

Office européen des brevets

(11) Numéro de publication: **0 216 642 B1**

# FASCICULE DE BREVET EUROPEEN

(45) Date de publication de fascicule du brevet:
**14.08.91**

(51) Int. Cl.⁵: **A61B 5/00, G01N 21/00**

(21) Numéro de dépôt: **86400919.6**

(22) Date de dépôt: **25.04.86**

(54) **Procédé pour mesurer une quantité de produit gras se trouvant à la surface d'un élément à étudier et appareillage pour sa mise en oeuvre.**

(30) Priorité: **29.04.85 FR 8506481**

(43) Date de publication de la demande:
**01.04.87 Bulletin 87/14**

(45) Mention de la délivrance du brevet:
**14.08.91 Bulletin 91/33**

(84) Etats contractants désignés:
**BE CH DE FR GB IT LI NL**

(56) Documents cités:
**DE-A- 2 353 224**
**FR-A- 2 404 845**
**FR-A- 2 480 461**
**US-A- 4 313 393**

**PATENT ABSTRACTS OF JAPAN, vol. 6, no. 175 (P-141)(1053), 9 septembre 1982 & JP A 57 90 161**

(73) Titulaire: **L'OREAL**
**14, Rue Royale**
**F-75008 Paris(FR)**

(72) Inventeur: **Yquel, Jean-Pierre**
**134, rue d'Estienne d'Orves**
**F-92700 Colombes(FR)**
Inventeur: **Grollier, Jean-François**
**16 Bis, Boulevard Morland**
**F-75004 Paris(FR)**
Inventeur: **Krien, Paul**
**4 Allée des Fusains**
**F-91370 Verrieres le Buisson(FR)**

(74) Mandataire: **Michardière, Bernard et al**
**C/O CABINET PEUSCET 68, rue d'Hauteville**
**F-75010 Paris(FR)**

## Description

La présente invention a pour objet un procédé et un appareillage pour détecter et mesurer une quantité de produit gras se trouvant à la surface d'un élément à étudier, notamment de la peau d'un sujet vivant ou de ses poils ou cheveux. Ce produit gras peut être le sébum secrété par la peau ou les cheveux, ou bien un produit du type huile subsistant, après un certain temps sur une peau qui, après une application dudit produit huileux, n'en a absorbé qu'une partie.

Dans le préambule de DE-A- 3 213 944, est répertorié un certain nombre de methodes pour évaluer l'importance du film gras, qui est présent à la surface de la peau. Cette demande de brevet porte elle-même sur un procédé pour évaluer le film gras de surface de la peau, procédé suivant lequel on met en contact avec une surface de peau la face d'un support revêtue d'un adsorbant finement divisé, de préférence minéral, on rend visible les substances grasses par décomposition ou action de réactifs et on évalue le changement de couleur photographiquement ou photométriquement.

On connaît également, par "CA SELECTS-COSMETIC CHEMICAL, ISSUE 19, 1984, 101:68596 h", une méthode de mesure de la sécrétion de sébum, suivant laquelle on utilise un support multicouche que l'on applique sur la peau pour prélever le sébum. Ce support multicouche comprend une couche de polyéthylène cellulaire, une couche de papier, une couche de couleur noire contenant de l'acrylate d'éthyle et une couche de couleur blanche d'une épaisseur de 5 $\mu$m contenant de l'acrylate d'éthyle. Lorsqu'on appuie ce support multicouche sur la peau, la couleur noire qui apparait sur la couche de couleur blanche est fonction de la quantité de sébum sécrété.

Parmi toutes les méthodes connues, on retiendra celles qui consistent à choisir, comme élément de prélèvement du sébum sur la peau, une plaque translucide, et à mesurer la quantité de lumière transmise au travers de ce support. On sait, en effet, qu'il existe une bonne corrélation entre, d'une part, la transparence d'une lame dépolie préalablement appliquée sur la zone de peau à étudier pendant un temps et avec une pression déterminés, et, d'autre part, la quantité de produit gras déposée sur ladite lame.

En particulier, FR-A-2 480 461 décrit un appareil destiné au repérage de la quantité de produit gras se trouvant à la surface de la peau, cet appareil mettant en oeuvre, dans un mode de réalisation préféré, un élément translucide constitué par une plaque de verre dépoli comportant une face arrière métallisée réfléchissante. La plaque est appliquée sur la peau par sa face non réfléchissante ; plus la sécrétion de sébum de la peau étudiée est importante, plus la plaque devient transparente. On envoie par une photodiode un flux lumineux monochromatique sur la face dépolie de la plaque et on récupère ce flux lumineux, après une première traversée de l'épaisseur de la plaque, puis une réflexion sur la face métallisée, et enfin une deuxième traversée de l'épaisseur de la plaque, sur la base d'un phototransistor qui fournit une tension sensiblement proportionnelle au flux lumineux reçu. Il existe une relation entre la quantité de sébum déposée sur la plaque et la tension à la sortie du phototransistor. Dans cette technique, la quantité de sébum agit sur la transmission du flux lumineux à travers le support utilisé.

DE-A-2 353 224 décrit un appareil et un procédé pour repérer la quantité de produit gras se trouvant à la surface de la peau, selon lequel une zone d'un ruban de matière plastique transparente, située devant un miroir, est appliquée contre la peau. La mesure porte sur la lumière traversant ladite zone et le ruban, réfléchie par le miroir, et traversant à nouveau le ruban.

Le document JP-A-57 90161 concerne une méthode de mesure d'une quantité de produit gras à la surface de la peau, méthode selon laquelle on fait adhérer le produit gras à un ruban transparent présentant une face rugueuse et on effectue une mesure de la quantité de produit gras à partir de la lumière transmise, ou de la lumière réfléchie par la surface sur laquelle a adhéré le produit gras. Ce document enseigne de travailler par transmission ou par réflexion, c'est-à-dire qu'il considère comme équivalents ces deux types de mesure. Ce document n'a pas mis en évidence les caractéristiques de l'invention qui permettent de tirer avantage au maximum de la méthode de mesure par réflexion sur la surface imprégnée.

La société déposante a constaté que la détection de la quantité de produit gras prélevé sur la peau ou les cheveux et déposé sur un support, par mesure, non plus de la quantité de lumière transmise à travers le support, mais de la quantité de lumière réfléchie sur le support, dans les conditions définies ci-après, conduisait à une plus grande précision dans les résultats. Ce mode de détection par réflexion, associé à l'emploi d'un support mat, offre l'avantage d'une plus grande dynamique de mesure que celle, associée aux mesures par transmission ou par réflexion sur un support brillant.

La présente invention a donc pour objet un procédé permettant de déterminer une quantité de produit gras se trouvant à la surface d'un élément à étudier, constitué notamment par la peau ou les cheveux d'un sujet vivant, procédé suivant lequel : on appuie une lame de prélèvement sur l'élément à étudier, pendant un temps donné et avec une pression donnée ; et on effectue l'évaluation de la quantité de lumière réfléchie par la face précitée de ladite lame, avant et après imprégnation par le produit gras ; et on déduit la quantité de produit gras se trouvant à la surface de l'élément étudié, caractérisé par le fait qu'on utilise une lame de prélèvement dont la face destinée à venir en contact avec l'élément à étudier est mate, et présente un coefficient de réflexion spéculaire inférieur à 1 %, et que pour l'évaluation de la quantité de lumière réfléchie par la face imprégnée de la lame, on pose la lame sur une surface de support non réfléchissante et absorbante à l'égard du rayonnement lumineux utilisé.

On choisit avantageusement une lame de prélèvement dont la face destinée à venir en contact avec l'élément à étudier présente un état de surface dont la finesse est donnée par une valeur de la rugosité arithmétique Ra inférieure à $0,50\mu m$, des microporosités, dont la capacité d'absorption d'un volume de fluide est inférieure à $0,25$ $mm^3/cm^2$, la profondeur de diffusion étant inférieure à $10\mu m$, cette dernière caractéristique permettant une mise en équilibre rapide du produit gras sur la lame de prélèvement.

Dans la définition ci-dessus, le volume des microporosités correspond au volume de liquide susceptible d'être absorbé à la surface de la lame de prélèvement et la valeur Ra est la valeur moyenne de la rugosité calculée par la formule :

$$Ra = \frac{1}{L}\int_0^L |y| \, dx$$

où y est l'altitude d'un point de la surface par rapport au plan moyen pris comme référence

$$(tel \ que \int_0^L y \, dx = o)$$

et L la longueur de l'échantillon.

On peut utiliser une lame de prélèvement flexible ou rigide suivant le cas. Lorsque l'on étudie la sécrétion du sébum des cheveux, on utilise une lame de prélèvement flexible qui permet d'épouser la courbure du cuir chevelu ; dans le cas de cette utilisation, on peut avantageusement utiliser une lame de prélèvement rectangulaire de 7cm x 1cm, dont la flexibilité est telle qu'elle prenne, en son centre, une flèche de 10 à 15 mm sous la charge d'un poids de 200 g appliqué en son centre.

Egalement, on peut utiliser une lame de prélèvement translucide ou opaque. Cette lame de prélèvement peut être blanche, noire ou colorée.

Conformément à une caractéristique particulièrement intéressante du procédé selon la présente invention, on mesure la quantité de lumière réfléchie par la lame avant et après imprégnation par le produit gras en fonction de l'emplacement dudit produit gras sur cette lame. En d'autres termes, la lecture en réflexion tout le long de la lame donne une courbe de brillance en fonction de l'emplacement sur la lame. Ce mode de réalisation est particulièrement intéressant lorsque l'on étudie la sécrétion du sébum des cheveux, la courbe reflétant alors l'aspect gras du cheveu depuis sa racine jusqu'à sa pointe si la lame a été appliquée sur le cheveu de la racine à la pointe.

Bien entendu, il est possible dans une variante simplifiée du procédé selon l'invention, d'observer à l'oeil nu la lame après imprégnation par le produit gras, pour en tirer des conclusions qualitatives sur la charge en produit gras de l'élément à étudier.

La présence d'un produit gras sur une lame colorée conduit à une modification de l'intensité (au sens de Munsell) de sa couleur. On pourra avantageusement choisir une couleur suffisamment foncée, l'oeil étant plus sensible à une variation de l'intensité lorsque la couleur est sombre. A cet égard, dans l'échelle des intensités selon Munsell, dans laquelle l'intensité du noir est égale à 1 et celle du blanc égale à 9, le choix d'une couleur dont l'intensité est inférieure à 6, conduit à de bons résultats.

Il est également intéressant d'utiliser une lame de prélèvement magnétisable susceptible d'être positionnée dans la zone d'examen optique par un aimant ; cette technique permet une détection aisée de sa brillance, comme cela sera décrit ci-après. Une telle fixation magnétique peut notamment être utilisée quand la lame de prélèvement est un tronçon de bande magnétique souple.

La présente invention a également pour objet un appareil pour déterminer une quantité de produit gras se trouvant à la surface d'un élément à étudier, constitué notamment par la peau ou les cheveux d'un sujet vivant, comportant : une source de lumière directive produisant un faisceau lumineux destiné à éclairer une lame de prélèvement qui a été appuyée sur l'élément à étudier, pendant un temps donné et avec une pression donnée ; un dispositif détecteur de la quantité de lumière réfléchie par la face de la lame destinée à venir en contact avec la peau, avant et après imprégnation du produit gras ; et un dispositif de traitement du signal de sortie dudit dispositif détecteur, caractérisé par le fait que la face de cette lame destinée à venir en contact avec l'élément à étudier est mate et présente un coefficient de réflexion spéculaire inférieur à 1 % ; et qu'il comporte une surface de support non réfléchissante et absorbante à l'égard du rayonnement lumineux utilisé, surface contre laquelle est posée la lame.

Dans le cas où l'on réalise une lecture tout le long de la lame pour obtenir une courbe de réflexion, image de la répartition du produit gras sur la surface étudiée, l'appareil selon la présente invention comporte un support pour ladite lame, ledit support étant déplaçable parallèlement au plan de cette dernière.

Avantageusement, le dispositif de traitement du signal de sortie du détecteur est constitué par un convertisseur analogique/digital associé à une calculatrice programmable.

Pour mieux mettre en évidence l'intérêt de la présente invention, on va décrire ci-après une expérience comparative entre les modes de détection de la lumière réfléchie par une lame de prélèvement et de la lumière transmise au travers de cette lame, et pour mieux faire comprendre l'objet de l'invention, on va en décrire, à titre d'exemple purement illustratif et non limitatif, un mode de réalisation représenté sur le dessin annexé.

Sur ce dessin :

- la figure 1 est une vue schématique d'un dispositif expérimental utilisé pour la comparaison des modes de détection par transmission et par réflexion ;
- les figures 2 et 3 représentent chacune un montage électronique utilisé comme détecteur, pour mesurer la lumière reçue après transmission ou réflexion ;
- la figure 4 est une vue schématique d'un appareillage pour la mise en oeuvre de l'invention ;
- la figure 5 montre, en coupe transversale, le dispositif de positionnement d'une lame de prélèvement sur un support translatable de l'appareillage de la figure 4.

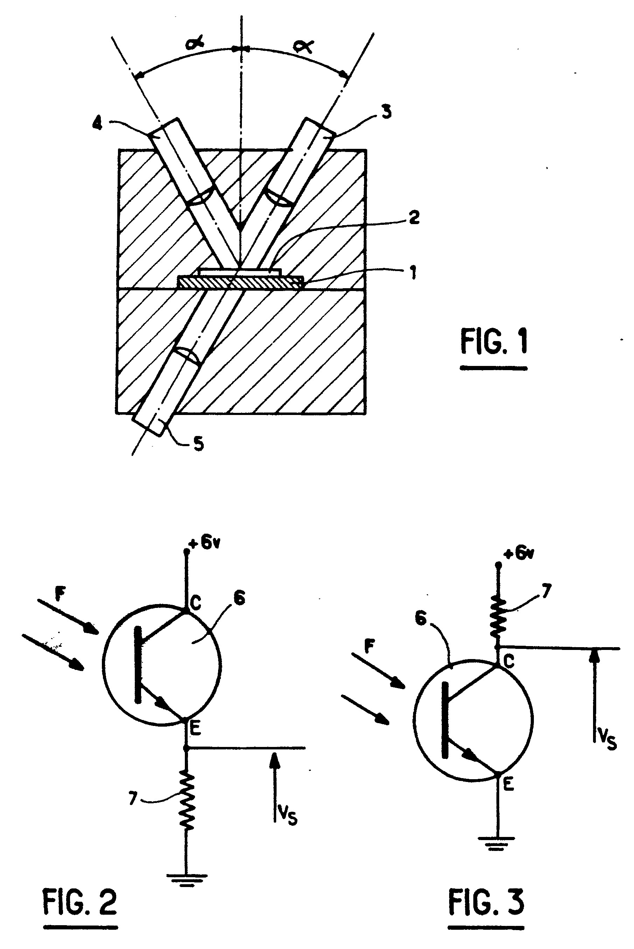

Sur la figure 1, on a représenté, en coupe transversale, une lame 1, sur la face supérieure de laquelle peut se trouver un dépôt de produit gras 2. La face de la lame 1, sur laquelle peut être déposé le produit 2, est mate. La lame 1 est éclairée par une source lumineuse 3 constituée par une lampe du type "Midget", alimentée par une tension continue de 7,5 volts. Cette lampe 3 fournit un faisceau lumineux incliné d'un angle $\propto = 30°$ par rapport à la normale au plan de la lame 1. On détecte en 4, selon un axe symétrique de l'axe d'éclairement par rapport à ladite normale, la quantité de lumière réfléchie par la lame 1, et en 5, selon un axe qui prolonge l'axe d'éclairement, la quantité de lumière transmise au travers de ladite lame 1.

Pour effectuer la comparaison sus-indiquée, on a réalisé des mesures sur deux lames 1 réalisées en des matériaux relativements transparents. La première est une lame de verre dépoli de 1 mm d'épaisseur et la seconde est un tronçon de ruban adhésif vendu sous la dénomination commerciale de "Scotch Magic", d'une épaisseur de 0,07 mm.

Dans une première série de mesure, on a utilisé le même circuit de détection en 4 et en 5, à savoir celui représenté sur la figure 2. Le détecteur est constitué par un phototransistor 6 du type "ASEA 2B50 C" dont le collecteur est alimenté à 6 volts; la base de ce phototransistor est irradiée par le flux lumineux F réfléchi ou transmis. Entre la masse et l'émetteur E, se trouve une résistance 7 de 10 k$\Omega$. Le signal mesuré $V_s$ est la différence de potentiel aux bornes de la résistance 7 . $V_s$ augmente quand l'irradiation du phototransistor 7 augmente.

Dans le cas de chacune des lames précitées, on a effectué trois mesures de $V_s$ en réflexion et en transmission, à chaque fois avant et après l'application de sébum; on a désigné par A et B les valeurs obtenues respectivement sans et avec sébum. Le dépôt de sébum est obtenu en appliquant la lame sur le front d'une personne pendant un temps donné et avec une pression donnée. La sensibilité est donnée par le rapport (B-A)/A.

Les résultats sont rapportés dans le tableau I ci-après. La comparaison des résultats dans les deux cas montre

TABLEAU I

| SUPPORT | REFLEXION | | | TRANSMISSION | | |
|---|---|---|---|---|---|---|
| | Sans Sébum (A) | Avec Sébum (B) | Sensibilité (B-A)/A | Sans Sébum (A) | Avec Sébum (B) | Sensibilité (B-A)/A |
| Verre dépoli | 0,08 | 0,90 | 10,25 | 4,10 | 5,90 | 0,44 |
| | 0,09 | 0,70 | 6,8 | 4,70 | 5,96 | 0,27 |
| | 0,13 | 0,70 | 4,4 | 4,6 | 5,50 | 0,20 |
| Scotch Magic | 0,10 | 0,90 | 8 | 5,96 | 6,02 | 0,01 |
| | 0,15 | 0,60 | 3 | 5,95 | 6,00 | 0,008 |
| | 0,16 | 0,50 | 2,1 | 5,98 | 6,00 | 0,003 |

clairement que le signal transmis par réflexion apparaît comme le plus sensible à la présence du sébum sur une lame de prélèvement.

Dans une deuxième série de mesures, la société déposante a cherché à augmenter la sensibilité du détecteur de la lumière transmise au travers de la lame sans modifier le détecteur de la lumière réfléchie; à cet effet, on a utilisé, pour la détection en 5 seulement, le montage électronique représenté sur la figure 3.

Ce montage diffère du précédent par le fait que l'émetteur est directement relié à la masse et qu'une résistance 7, de même valeur que la précédente, est reliée non plus à l'émetteur, mais au collecteur; la tension d'alimentation du collecteur est toujours de 6 Volts. On mesure, dans ce cas, le signal $V_s$, qui est la différence de potentiel entre le collecteur et la masse. $V_s$ diminue quand l'irradiation augmente.

Les mesures et les calculs effectués , analogues aux précédents, sont reportés dans le tableau II ci-après.

TABLEAU II

| SUPPORT | REFLEXION | | | TRANSMISSION | | |
|---|---|---|---|---|---|---|
| | Sans Sébum (A) | Avec Sébum (B) | Sensibilité (B-A)/A | Sans Sébum (A) | Avec Sébum (B) | Sensibilité (B-A)/A |
| Verre dépoli | 0,09 | 0,98 | 9,9 | 2,70 | 1,00 | - 0,63 |
| | 0,10 | 0,93 | 8,3 | 2,80 | 1,54 | - 0,45 |
| | 0,15 | 0,95 | 5,3 | 2,70 | 1,20 | - 0,56 |
| Scotch Magic | 0,05 | 0,25 | 4 | 1,2 | 0,22 | - 0,82 |
| | 0,06 | 0,40 | 5,7 | 1 | 0,2 | - 0,80 |
| | 0,09 | 0,50 | 4,6 | 1,8 | 0,20 | - 0,89 |

Bien que l'on ait utilisé, pour la détection par transmission au travers de la lame, un détecteur de plus grande sensibilité, la sensibilité de la détection par transmission reste encore bien inférieure à celle obtenue dans le cas de la détection par réflexion.

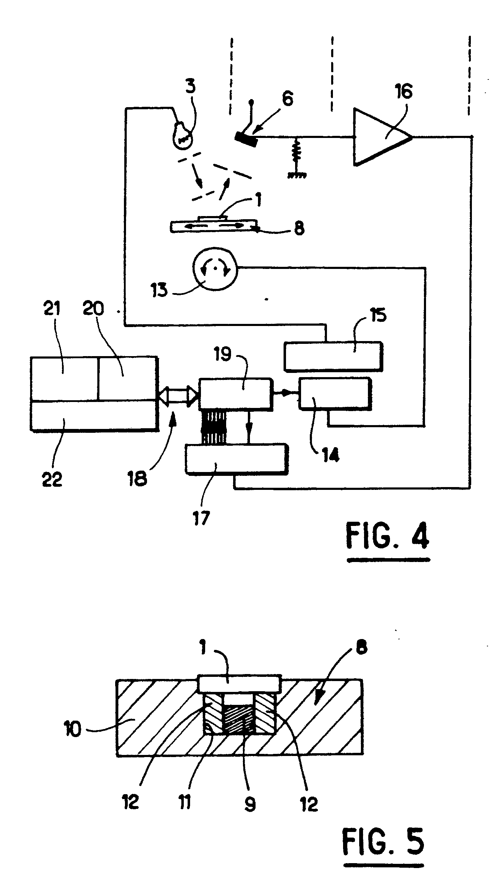

L'appareil représenté sur la figure 4 permet de déterminer la quantité de lumière réfléchie par une lame porteuse de sébum dans les zones où le sébum se trouve sur cette lame. Cet appareil est particulièrement intéressant dans le cas où l'on souhaite analyser la répartition du sébum le long des cheveux, entre leur racine et leur pointe; on peut en effet, obtenir une courbe représentant la distribution du sébum le long des cheveux. Pour ce faire, on applique sur les cheveux, une lame 1 rectangulaire souple, en exerçant une pression constante de la racine vers la pointe des cheveux, la flexibilité de la lame 1, permettant à celle-ci d'épouser le galbe du crâne. On peut avantageusement choisir, pour constituer une telle lame 1, un tronçon de bande magnétique, éventuellement monté sur une plaquette souple.

L'appareil de la figure 4 comporte un support 8 de la lame 1. La figure 5 représente une coupe dudit support. Le support 8 est constitué par un banc de glissement translatable 10, dans lequel est pratiquée une rainure 11 de section en U; dans le fond de la rainure 11, on dispose un aimant 9 entre deux barres rectifiées 12, sur lesquelles la lame 1 est en appui par sa face opposée à celle devant porter le produit gras. Sur cette lame 1 sont couchées des particules d'oxyde de fer, de sorte que la lame 1 est soumise à l'attraction magnétique de l'aimant 9 et, fixée de façon plane sur le support 8.

La surface du support 8, ou une surface intermédiaire, contre ou au-dessus de laquelle est posée la lame 1, est non réfléchissante et absorbante à l'égard du rayonnement lumineux utilisé. On évite ainsi une réflexion parasite de la lumière qui aurait pu traverser la lame 1.

Le déplacement du support 8 est assuré au moyen d'une vis sans fin par un moteur pas à pas 13, dont la commande est schématisé en 14.

L'appareil de la figure 4 comporte également une lampe 3, telle que décrite pour le dispositif de la figure 1; cette lampe est alimentée par le circuit 15; l'appareil comporte, en outre, un dispositif de détection constitué par un phototransistor 6, dont le montage est représenté sur la figure 2 et a déjà été décrit.

On sait que la présence du sébum sur la lame 1 modifie le pouvoir de réflexion, c'est-à-dire la brillance, de cette lame; le phototransistor 6 fournit un signal $V_s$, dont la valeur est corrélée à la quantité de sébum présente sur la lame 1, ce signal $V_s$ étant indépendant de la répartition transversale du sébum sur la lame. Le signal $V_s$ provenant du phototransistor 6 est amplifié grâce à l'amplificateur 16, puis numérisé dans le convertisseur analogique/digital 17, qui transforme le signal dont l'amplitude est comprise entre 0 et 10 Volts en une valeur lue par une calculatrice programmable 18 comportant une unité centrale à interface 19, une imprimante 20, un écran 21 et un clavier 22. La calculatrice 18 a pour rôle d'assurer et de contrôler le déplacement du support 8 par rapport à la tête de lecture constituée par la lampe 3 et le détecteur 6, de mémoriser les données provenant du détecteur 6 en fonction de la position de la lame 1 par rapport à celui-ci, et de traiter ces données avant d'éditer un rapport contenant différentes informations concernant la répartition du sébum le long de la lame 1; ces informations sont notamment un graphique représentant le spectre de la répartition du sébum le long de la lame, la valeur de la surface du spectre, la hauteur du pic, la largeur du spectre à mi-hauteur, etc... La courbe représentant la distribution du sébum le long des cheveux est issue de la différence entre le spectre obtenu avec la lame 1 portant le sébum et le spectre obtenu avec la lame 1 seule, avant application de sébum.

Il est bien entendu que le mode de réalisation ci-dessus décrit n'est aucunement limitatif et pourra donner lieu à toutes modifications désirables, sans sortir pour cela du cadre de la présente invention.

## Revendications

1. Procédé permettant de déterminer une quantité de produit gras se trouvant à la surface d'un élément à étudier, constitué notamment par la peau ou les cheveux d'un sujet vivant, procédé suivant lequel : on appuie une lame de prélèvement (1) sur l'élément à étudier, pendant un temps donné et avec une pression donnée ; et on effectue l'évaluation de la quantité de lumière réfléchie par la face précitée de ladite lame, avant et après imprégnation par le produit gras ; et on déduit la quantité de produit gras se trouvant à la surface de l'élément étudié, caractérisé par le fait qu'on utilise une lame de prélèvement (1) dont la face destinée à venir en contact avec l'élément à étudier est mate, et présente un coefficient de réflexion spéculaire inférieur à 1 %, et que pour l'évaluation de la quantité de lumière réfléchie par la face imprégnée de la lame, on pose la lame sur une surface de support non réfléchissante et absorbante à l'égard du rayonnement lumineux utilisé.

2. Procédé selon la revendication 1, caractérisé par le fait qu'on utilise une lame de prélèvement (1), dont la face destinée à venir en contact avec l'élément à étudier présente un état de surface ayant une finesse correspondant à une valeur de Ra inférieure à 0,50 µm.

3. Procédé selon la revendication 1 ou 2, caractérisé par le fait qu'on utilise une lame de prélèvement (1), dont la face destinée à venir en contact avec l'élément à étudier présente des microporosités, dont la capacité d'absorption d'un fluide est inférieure à 0,25 mm³/cm², la profondeur de diffusion étant inférieure à 10 µm.

4. Procédé selon l'une des revendications 1 à 3, caractérisé par le fait qu'on utilise une lame de prélèvement (1) flexible.

5. Procédé selon l'une des revendications 1 à 3, caractérisé par le faitqu'on utilise une lame de prélèvement (1) rigide.

6. Procédé selon l'une des revendications 1 à 5, caractérisé par le fait qu'on utilise une lame de prélèvement (1) translucide.

7. Procédé selon l'une des revendications 1 à 5, caractérisé par le fait qu'on utilise une lame de prélèvement (1) opaque.

8. Procédé selon l'une des revendications 1 à 7, caractérisé par le fait qu'on utilise une lame de prélèvement (1) colorée.

9. Procédé selon l'une des revendications 1 à 8, caractérisé par le fait qu'on utilise une lame de prélèvement (1) ayant des caractéristiques magnétiques permettant son positionnement et sa fixation par un aimant.

10. Procédé selon l'une des revendications 1 à 9, caractérisé par le fait qu'avant et après imprégnation par le produit gras, on mesure la quantité de lumière réfléchie par la lame (1) en fonction de l'emplacement sur la lame de prélèvement (1).

11. Appareil pour déterminer une quantité de produit gras se trouvant à la surface d'un élément à étudier, constitué notamment par la peau ou les cheveux d'un sujet vivant, comportant :
    - une source de lumière directive (3) produisant un faisceau lumineux (F) destiné à éclairer une lame de prélèvement (1) qui a été appuyée sur l'élément à étudier, pendant un temps donné et avec une pression donnée ;
    - un dispositif détecteur (6) de la quantité de lumière réfléchie par la face de la lame (1) destinée à venir en contact avec la peau, avant et après imprégnation du produit gras ;
    - et un dispositif de traitement du signal de sortie dudit dispositif détecteur (6);
    caractérisé par le fait que la face de cette lame (1) destinée à venir en contact avec l'élément à étudier est mate et présente un coefficient de réflexion spéculaire inférieur à 1 % ; et qu'il comporte une surface de support (8) non réfléchissante et absorbante à l'égard du rayonnement lumineux utilisé, surface contre laquelle est posée la lame (1).

12. Appareil selon la revendication 11, caractérisé par le fait que le support (8) pour ladite lame de prélèvement (1), est déplaçable dans un plan parallèle à celui de ladite lame (1).

13. Appareil selon l'une des revendications 11 et 12, caractérisé par le fait que le dispositif de traitement du signal de sortie du détecteur (6) comporte un convertisseur analogique/digital (17) associé à une calculatrice programmable (18).

**Claims**

1. A method allowing a quantity of a greasy substance found on the surface of an element to be investigated, constituted in particular by the skin or hair of a living subject, according to which method: a take-up strip (1) is applied to the element to be investigated for a given time and with a given pressure; and the quantity of light reflected by the aforesaid surface of the said strip is evaluated before and after impregnation with the greasy substance; and the quantity of the greasy substance located on the surface of the investigated element is derived, characterized in that a take-up strip (1) is used

EP 0 216 642 B1

whose surface intended to come into contact with the element to be investigated is matt and has a specular reflection factor of less than 1%, and in that for the evaluation of the quantity of light reflected by the impregnated strip surface, the strip is placed on a bearing surface which is nonreflective and absorbent with respect to the luminous radiation used.

2. A method according to claim 1, characterized in that a take-up strip (1) is used, whose side intended to come into contact with the element to be investigated has a surface condition with a smoothness corresponding to an Ra value of less than 0.50 $\mu$m.

3. A method according to claim 1 or 2, characterized in that a take-up strip (1) is used whose side intended to come into contact with the element to be investigated has microporosities whose fluid absorption capacity is less than 0.25 mm$^3$/cm$^2$, the depth of diffusion being less than 10 $\mu$m.

4. A method according to one of claims 1 to 3, characterized in that a flexible take-up strip (1) is used.

5. A method according to one of claims 1 to 3, characterized in that a rigid take-up strip (1) is used.

6. A method according to one of claims 1 to 5, characterized in that a translucent take-up strip (1) is used.

7. A method according to one of claims 1 to 5, characterized in that an opaque take-up strip (1) is used.

8. A method according to one of claims 1 to 7, characterized in that a coloured take-up strip (1) is used.

9. A method according to one of claims 1 to 8, characterized in that a take-up strip (1) is used, having magnetic characteristics allowing it to be positioned and fixed by a magnet.

10. A method according to one of claims 1 to 9, characterized in that before and after impregnation with the greasy substance, the quantity of light reflected by the take-up strip (1) is measured as a function of the location on the take-up strip (1).

11. An apparatus for determining a quantity of a greasy substance found on the surface of an element to be investigated, constituted in particular by the skin or hair of a living subject comprising:
    - a directional light source (3) producing a luminous beam F intended to illuminate a take-up strip (1) which has been applied to the element to be investigated, for a given time and with a given pressure,
    - a device (6) for detecting the quantity of light reflected by the side of the strip (1) intended to come into contact with the skin, before and after impregnation with the greasy substance;
    - and a device for processing the output signal of the said detector device (6);
    - characterized in that the side of this strip (1) intended to come into contact with the element to be investigated is matt and has a specular reflection factor of less than 1%; and that it comprises a nonreflective and absorbent bearing surface (8) with respect to the luminous radiation used, against which surface the strip (1) is positioned.

12. An apparatus accoording to claim 11, characterized in that the support (8) for the said take-up strip (1) is displaceable in a plane parallel to that of the said strip (1).

13. An apparatus according to one of claims 11 and 12, characterized in that the device for processing the output signal of the detector (6) comprises an analog/digital converter (17) associated with a programmable computer.

**Patentansprüche**

1. Verfahren zur quantitativen Bestimmung eines fettartigen Produkts, das sich auf der Oberfläche einer zu untersuchenden Probe, insbesondere auf der Haut oder den Haaren einer lebenden Person, befindet, wobei man ein Entnahmeplättchen (1) während einer vorgegebenen Zeitspanne und mit einem vorgegebenen Druck auf die zu untersuchende Probe aufdrückt; die von dieser Seite des Plättchens reflektierte Lichtmenge vor und nach Imprägnierung mit dem fettartigen Produkt bestimmt; und die Menge des fettartigen Produkts, die sich auf der Oberfläche der untersuchten Probe befindet,

9

ableitet,

**dadurch gekennzeichnet, daß**

man ein Entnahmeplättchen (1) einsetzt, dessen Seite, die mit der zu untersuchenden Probe in Kontakt kommt, matt ist und einen Reflexionskoeffizienten kleiner als 1% besitzt, und
**daß** man zur Bestimmung der Lichtmenge,
die von der imprägnierten Seite des Plättchens reflektiert wird, das Plättchen auf eine nichtreflektierende und für die verwendete Lichtstrahlung absorbierende Oberfläche eines Halters auflegt.

2. Verfahren nach Anspruch 1,

**dadurch gekennzeichnet, daß**

man ein Entnahmeplättchen (1) einsetzt, dessen Seite, die mit der zu untersuchenden Probe in Kontakt kommt, eine Oberflächenbeschaffenheit mit einer Feinheit besitzt, die einem Ra-Wert kleiner als 0,50 $\mu$m entspricht.

3. Verfahren nach Anspruch 1 oder 2,

**dadurch gekennzeichnet, daß**

man ein Entnahmeplättchen (1) einsetzt, dessen Seite, die mit der zu untersuchenden Probe in Kontakt kommt, Mikroporen aufweist , deren Absorptionskapazität für ein Fluid geringer als 0,25 mm$^3$/cm$^2$ ist, wobei die Diffusionstiefe kleiner als 10$\mu$m ist.

4. Verfahren nach einem der Ansprüche 1 bis 3,

**dadurch gekennzeichnet, daß**

man ein flexibles Entnahmeplättchen (1) einsetzt.

5. Verfahren nach einem der Ansprüche 1 bis 3,

**dadurch gekennzeichnet, daß**

man ein steifes Entnahmeplättchen (1) einsetzt.

6. Verfahren nach einem der Ansprüche 1 bis 5,

**dadurch gekennzeichnet, daß**

man ein durchsichtiges Entnahmeplättchen (1) einsetzt.

7. Verfahren nach einem der Ansprüche 1 bis 5,

**dadurch gekennzeichnet, daß**

man ein opakes Entnahmeplättchen (1) einsetzt.

8. Verfahren nach einem der Ansprüche 1 bis 7,

**dadurch gekennzeichnet, daß**

man ein gefärbtes Entnahmeplättchen (1) einsetzt.

9. Verfahren nach einem der Ansprüche 1 bis 8,

**dadurch gekennzeichnet, daß**

man ein Entnahmeplättchen (1) einsetzt, das magnetische Eigenschaften besitzt, die dessen Positionierung und Fixierung mit Hilfe eines Magneten ermöglichen.

10. Verfahren nach einem der Ansprüche 1 bis 9,

**dadurch gekennzeichnet, daß**

man vor und nach dem Imprägnieren mit dem fettartigen Produkt die von dem Entnahmeplättchen (1) reflektierte Lichtmenge in Abhängigkeit von dem Ort auf dem Entnahmeplättchen (1) bestimmt.

11. Vorrichtung zur quantitativen Bestimmung eines fettartigen Produkts, das sich auf der Oberfläche einer zu untersuchenden Probe, insbesondere auf der Haut oder den Haaren einer lebenden Person, befindet, die
   - eine gerichtete Lichtquelle (3), die einen Lichtstrahl F zum Beleuchten eines Entnahmeplättchens (1) liefert, welches während einer vorgegebenen Zeitspanne und mit einem vorgegebenen Druck auf die zu untersuchende Probe aufgedrückt wurde;
   - eine Detektorvorrichtung (6) zur Bestimmung der Lichtmenge, die vor und nach dem Imprägnieren mit dem fettartigen Produkt von der Seite des Entnahmeplättchens (1) reflektiert wird, die mit der Haut in Kontakt kommt; und
   - eine Vorrichtung zum Verarbeiten des Ausgangssignals dieser Detektorvorrichtung (6),
   aufweist,
   **dadurch gekennzeichnet, daß**
   die Seite des Entnahmeplättchens (1), die mit der Oberfläche der zu untersuchenden Probe in Kontakt kommt, matt ist und einen Reflexionskoeffizienten kleiner als 1 % besitzt; und die Vorrichtung eine nicht reflektierende und für die verwendete Lichtstrahlung absorbierende Oberfläche eines Halters (8) aufweist, an die das Plättchen (1) angelegt wird.

12. Vorrichtung nach Anspruch 11,

**dadurch gekennzeichnet, daß**

der Halter (8) für das Entnahmeplättchen (1) in einer Ebene verschiebbar ist, die parallel zu der des Plättchens (1) verläuft.

13. Vorrichtung nach Anspruch 11 oder 12,

**dadurch gekennzeichnet, daß**

die Vorrichtung zum Verarbeiten des Ausgangssignals des Detektors (6) einen Analog-/Digitalumwandler (17) besitzt, der mit einem Rechenprogramm (18) gespeist ist.

FIG. 1

FIG. 2

FIG. 3

FIG. 4

FIG. 5

13